# EUROPEAN PATENT SPECIFICATION

(11) **EP 2 066 345 B1**
(45) Date of publication and mention of the grant of the patent: **25.02.2015**
(21) Application number: 07825694.8
(22) Date of filing: 11.09.2007
(51) Int. Cl.: A61K 39/145

(54) **MAKING INFLUENZA VIRUS VACCINES WITHOUT USING EGGS**
HERSTELLUNG VON INFLUENZA-VIRUS-IMPFSTOFFEN OHNE VERWENDUNG VON EIERN
FABRICATION DE VACCINS CONTRE LE VIRUS GRIPPAL SANS UTILISER D'OEUFS

(30) Priority: 11.09.2006 US 843720 P; 18.06.2007 US 936279 P
(43) Date of publication of application: 10.06.2009
(60) Divisional application: 12156030.4
(73) Proprietor: Novartis AG, 4056 Basel (CH)
(72) Inventor: TSAI, Theodore F., Philadelphia, PA 19103 (US); TRUSHEIM, Heidi, 35041 Marburg (DE)
(74) Representative: Marshall, Cameron John
(86) International application number: PCT/IB2007/003536
(87) International publication number: WO 2008/032219

(56) References cited:
- WO-A-03/004049
- WO-A-2006/027698
- US-A- 5 631 350
- US-B1- 6 344 354
- MEDEMA JEROEN K ET AL: "Modeling pandemic preparedness scenarios: health economic implications of enhanced pandemic vaccine supply." VIRUS RESEARCH, vol. 103, no. 1-2, July 2004 (2004-07), pages 9-15, XP002477504 ISSN: 0168-1702 cited in the application
- MERTEN O W ET AL: "PRODUCTION OF INFLUENZA VIRUS IN CELL CULTURES FOR VACCINE PREPARATION" ADVANCES IN EXPERIMENTAL MEDICINE AND BIOLOGY, vol. 397, 1996, pages 141-151, XP000982880 ISSN: 0065-2598 cited in the application
- HANSON BRENDON J ET AL: "Passive immunoprophylaxis and therapy with humanized monoclonal antibody specific for influenza A H5 hemagglutinin in mice" RESPIRATORY RESEARCH, vol. 7, no. 1, 14 October 2006 (2006-10-14), page 126, XP021021469 ISSN: 1465-9921
- PALLADINO G ET AL: "Virus-neutralizing antibodies of immunoglobulin G (IgG) but not of IgM or IgA isotypes can cure influenza virus pneumonia in SCID mice." JOURNAL OF VIROLOGY, vol. 69, no. 4, April 1995 (1995-04), pages 2075-2081, XP002493444 ISSN: 0022-538X
- HORIMOTO ET AL: "The development and characterization of H5 influenza virus vaccines derived from a 2003 human isolate" VACCINE, vol. 24, no. 17, 24 April 2006 (2006-04-24), pages 3669-3676, XP005353720 ISSN: 0264-410X

## Description

### TECHNICAL FIELD

This invention is in the field of manufacturing vaccines for protecting against influenza virus.

### BACKGROUND ART

The current process for preparing seasonal vaccines against human influenza virus infection involves the following steps [1,2]: (a) isolation of circulating virus strains; (b) antigenic and genetic analysis of isolated viruses; (c) selection of viral strains for use during the coming season; (d) preparation of high-growth seed strains by reassortment or the use of reverse genetics; (e) release of seed strains to vaccine manufacturers; (f) evaluation by the manufacturers of the strains' suitability for industrial production; and (g) growth of the seed strains to produce virus from which vaccines are then manufactured.

Steps (a) to (e) of this process are performed by the FDA and government-approved international influenza centres, typically under the auspices of the World Health Organisation; steps (f) and (g) are performed by the manufacturers themselves.

Step (d) transitions a virus from one that is naturally adapted for infecting humans into one that will grow to high titers under industrial growth conditions. For influenza A virus, this step typically involves creating a 6:2 reassortant strain that includes the HA- and NA-encoding genome segments from the strains selected in (c) and the remaining six genome segments from a strain that grows efficiently in chicken eggs, and this strain is usually A/PR/8/34. The reassortment procedure is then followed by repeated passaging of the strain in embryonated eggs to allow for egg adaptation and growth enhancement. For influenza B virus, prototype strains with good growth characteristics are usually obtained by direct and repeated passaging in embryonated eggs without attempting to generate reassortants.

Thus the steps performed prior to release to vaccine manufacturers involve passaging influenza virus through eggs. Even if the viruses are grown by a manufacturer in step (g) on a cell substrate, rather than on eggs, the virus will still have been passaged through eggs at some stage between isolation from in step (a) and receipt by a manufacturer in step (e).

For instance, step (a) involves exposing a substrate to a patient sample, such that any virus in the sample will infect the substrate. The substrate can then amplify the amount of virus present, and the amplified viruses are then available for further study. This step may take place in eggs or in mammalian cells. Cells known for use in primary isolation include MRC-5 cells [3], Vero cells [4,5], MDCK cells [6], HepG2 cells [7], LLC-MK2 cells [8], *etc.* In general, though, chicken eggs continue to be used to isolate reference strains for the manufacture of influenza vaccine. The use of eggs is so important to current procedures that in the 2003-04 season the FDA rejected use of the most appropriate H3N2 strain (A/Fujian/411/2002) because it had not originally been isolated in eggs [2,9] and no antigenically-similar egg-isolated strains were available.

It has previously been proposed to remove the use of eggs from various stages of influenza virus manufacture.

Reference 10 proposes that vaccines should be grown in cell culture using either a (i) a high growth strain of a passaged clinical isolate or (ii) a reassortant derived from at least one naturally-occurring mammalian influenza virus strain, provided that the isolate or reassortant has not been passaged in avian eggs. Thus the process described in reference 10 begins with a seed virus that has already been selected or manipulated for growth in the cell culture of choice.

Reference 11 compares viruses passaged through eggs with those passaged through MDCK cells, but specifically selects the former for vaccine manufacture.

Reference 12 suggests that seed viruses for pandemic influenza vaccines could be prepared by propagating the pandemic strain directly onto mammalian cell culture instead of via embryonated eggs, but notes that egg passaging was obligatory for inter-pandemic manufacture. The reason for this obligatory egg passaging is that it has been believed to act as a 'filter' for adventitious agents: regulatory agencies have accepted that a series of passages in an avian system, between the original clinical isolation from a human and the final vaccine for administration to a human, will prevent mammalian-type adventitious agents from co-replicating with the influenza virus.

The invention aims to provide further procedures useful in manufacturing influenza vaccines, in which the use of eggs is reduced, and preferably is avoided altogether. In one particular aspect, the invention aims to provide further and improved procedures useful in influenza virus isolation.

### DISCLOSURE OF THE INVENTION

Although it has previously been proposed to remove the use of eggs from various stages of influenza virus manufacture, the invention differs from these proposals in various aspects.

### Receptor binding

Human influenza viruses bind to receptor oligosaccharides having a Sia(α2,6)Gal terminal disaccharide (sialic acid linked α-2,6 to galactose), but eggs instead have receptor oligosaccharides with a Sia(α2,3)Gal terminal disaccharide. Growth of human influenza viruses in eggs provides selection pressure on hemagglutinin away from Sia(α2,6)Gal binding towards Sia(α2,3)Gal binding.

Like eggs, Vero cells express predominantly Sia(α2,3)Gal receptors [15]. In contrast, MDCK cells and PER.C6 cells express both Sia(α2,3)Gal and Sia(α2,6)Gal. Reference 16 reports transfection of MDCK cells to overexpress α-2,6-sialyltransferase in order to favour selection of Sia(α2,6)Gal binding. Even without such manipulations, however, it is possible to grow influenza viruses on MDCK cells without shifting them towards Sia(α2,3)Gal binding. Thus the invention can use cells that express both Sia(α2,3)Gal and Sia(α2,6)Gal, but can produce influenza viruses that have a binding preference for oligosaccharides with a Sia(α2,6)Gal terminal disaccharide compared to oligosaccharides with a Sia(α2,3)Gal terminal disaccharide.

In preferred embodiments of the first and second aspects of the invention, influenza viruses used for infection in step (i) have a binding preference for oligosaccharides with a Sia(α2,6)Gal terminal disaccharide compared to oligosaccharides with a Sia(α2,3)Gal terminal disaccharide. This binding preference is retained during step (ii) and step (iii), such that the influenza virus produced in step (iii) has a binding preference for oligosaccharides with a Sia(α2,6)Gal terminal disaccharide compared to oligosaccharides with a Sia(α2,3)Gal terminal disaccharide.

In preferred embodiments of the third and fourth aspects of the invention, influenza viruses used for infection in step (ii) have a binding preference for oligosaccharides with a Sia(α2,6)Gal terminal disaccharide compared to oligosaccharides with a Sia(α2,3)Gal terminal disaccharide. This binding preference is retained during step (iii) and step (iv), such that the influenza virus produced in step (iv) has a binding preference for oligosaccharides with a Sia(α2,6)Gal terminal disaccharide compared to oligosaccharides with a Sia(α2,3)Gal terminal disaccharide.

To determine if a virus has a binding preference for oligosaccharides with a Sia(α2,6)Gal terminal disaccharide compared to oligosaccharides with a Sia(α2,3)Gal terminal disaccharide, various assays can be used. For instance, reference 13 describes a solid-phase enzyme-linked assay for influenza virus receptor-binding activity which gives sensitive and quantitative measurements of affinity constants. Reference 14 used a solid-phase assay in which binding of viruses to two different sialylglycoproteins was assessed (ovomucoid, with Sia(α2,3)Gal determinants; and pig α₂-macroglobulin, which Sia(α2,6)Gal determinants), and also describes an assay in which the binding of virus was assessed against two receptor analogs: free sialic acid (Neu5Ac) and 3'-sialyllactose (Neu5Acα2-3Galβ1-4Glc). Reference 15 reports an assay using a glycan array which was able to clearly differentiate receptor preferences for α2,3 or α2,6 linkages. Reference 16 reports an assay based on agglutination of human erythrocytes enzymatically modified to contain either Sia(α2,6)Gal or Sia(α2,3)Gal. Depending on the type of assay, it may be performed directly with the virus itself, or can be performed indirectly with hemagglutinin purified from the virus.

### Reference materials

The current process for manufacturing influenza viruses involves the preparation of reference reagents for each strain, namely (i) an anti-HA sera and (ii) purified whole virions. These calibrated reagents are used in a SRID assay to determine the level of HA in bulk antigens produced by manufacturers, thereby allowing them to dilute the bulks to give vaccines with the desired amount of HA per dose.

With the current process, where reference strains have been passaged through eggs and production strains are optimised for growth in eggs, the sera and antigens in the reference reagents are well matched. It has been found, however, that the sera can be a poor match for antigens produced in cell culture, presumably because of the different selection pressures in the different systems. Poor reactivity between the reference sera and the antigen mean that HA levels will be under-estimated, leading to (i) fewer doses from a given bulk and (ii) over-dosing of HA in a vaccine.

To overcome the problem of matching antigens derived from cell culture with sera raised against egg-derived materials, the invention provides reference materials based on viruses that have not been adapted to egg-based growth.

Thus the invention provides in claim 1 a process for preparing an antiserum from a non-human animal, comprising steps of: (i) administering to the non-human animal a purified influenza virus hemagglutinin; and then (ii) recovering from the non-human animal serum containing antibodies that recognise the hemagglutinin, characterised in that the hemagglutinin used in step (i) is from a virus grown in a mammalian cell line.

The hemagglutinin used in step (i) is preferably from a virus that has never been grown in eggs. For example, the hemagglutinin may have a binding preference for oligosaccharides with a Sia(α2,6)Gal terminal disaccharide compared to oligosaccharides with a Sia(α2,3)Gal terminal disaccharide.

Preferred hemagglutinin used to raise the antiserum is glycosylated with glycans obtainable from growth in a mammalian cell line *(e.g.* the cell lines described herein), such as MDCK.

Antisera may be generated for influenza A viruses and influenza B viruses.

The animal is preferably a mammal, such as a goat or more preferably a sheep. Antisera can be conveniently prepared in sheep by extracting HA from purified virus by treatment with bromelain, followed by purification by sedimentation on a sucrose gradient. A dose of about 50µg hemagglutinin is administered intramuscularly to a sheep in combination with Freund's complete adjuvant (FCA). A 10µg dose can be given two weeks later, and then 2-4 further doses at weekly intervals. Thereafter, serum can be collected. Prior to use, it may be diluted (*e.g.* with PBS buffer containing sodium azide) and filled into containers. The serum may be exposed to an acidic pH *(e.g.* pH 5 for two hours) in order to meet foot and mouth disease regulations.

The invention also provides in claim 2 a process for preparing an antiserum from a non-human animal, comprising steps of: (i) growing influenza virus in a mammalian cell line; (ii) purifying hemagglutinin antigen from virus grown in step (i); (iii) administering the purified hemagglutinin from step (ii) to the non-human animal; and then (iv) recovering from the non-human animal serum containing antibodies that recognise the hemagglutinin.

Disclosed herein is an antiserum obtainable by these processes.

Disclosed herein is a gel including this antiserum. Thus a process as described above for preparing an antiserum from an animal may include the further step of mixing the antiserum with a gel. The gel is suitable for performing a SRID assay *e.g.* it is an agarose gel.

Disclosed herein are antigen reference materials. Thus the invention discloses a process for preparing an antigen reference material, comprising steps of: (i) growing influenza virus in a cell line; (ii) purifying viruses grown in step (i); and (iii) inactivating the virus, characterised in that the influenza virus used in step (i) has never been grown in eggs. The process may include a further step of: (iv) lyophilising the inactivated virus.

The virus used in step (i) has never been grown in eggs. For example, its hemagglutinin may have a binding preference for oligosaccharides with a Sia(α2,6)Gal terminal disaccharide compared to oligosaccharides with a Sia(α2,3)Gal terminal disaccharide.

The reference material is free from egg-derived materials (*e.g*. free from ovalbumin, free from ovomucoid, free from chicken DNA). Glycoproteins in the reference material will be glycosylated with glycans obtainable from growth in a mammalian cell line *(e.g.* the cell lines described herein), such as MDCK. Reference materials may be generated for influenza A viruses and influenza B viruses.

Reference materials are usually used in pairs, and so the invention also discloses a kit comprising: (i) antiserum obtainable by these processes and (ii) antigen reference material obtainable by these processes.

The invention also provides in claim 8 a process for preparing the kit, comprising the steps of: (i) making an antiserum as described above; (ii) making an antigen reference material as described above; and (iii) combining the products of steps (i) and (ii) into a kit.

The antigen and antiserum are suitable and intended for use in SRID assays, and the invention discloses a single radial immunodiffusion assay for influenza virus hemagglutinin, characterised in that the assay uses antiserum obtainable by these processes and/or antigen reference material obtainable by these processes. The SRID assay will involve steps of preparing a gel including the antiserum, applying the antigen reference material (reconstituted, where necessary, in an aqueous medium) to the gel (typically into a well), and then permitting the antigen to diffuse radially into the gel. The antigen may be treated with a detergent, such as a Zwittergent detergent, prior to use.

### Viruses (including seed viruses) prepared or isolated by techniques of the invention

Preferred influenza A viruses of the invention (including seed viruses, viruses isolated from patient samples using MDCK cells, reassortant viruses, *etc.)* include fewer than 6 *(i.e.* 0, 1, 2, 3, 4 or 5) viral segments from a PR/8/34 influenza virus. Preferably they include no PR/8/34 segments. If any PR/8/34 segment(s) is/are present then this/these will not include the PR/8/34 HA segment and usually will not include the PR/8/34 NA segment. Thus preferred viruses are those in which at least one of segments NP, M, NS, PA, PB1 and/or PB2 is not derived from PR/8/34. More preferably, at least one of segments NP, M, PA, PB1 and/or PB2 is not derived from PR/8/34. Thus the invention can improve over existing vaccines by adding to the normal HA and NA antigens one or more further epitope-containing antigens that is/are representative of a circulating strain.

Similarly, preferred influenza A viruses include fewer than 6 *(i.e.* 0, 1, 2, 3, 4 or 5) viral segments from an AA/6/60 influenza virus (A/Ann Arbor/6/60). Preferably they include no AA/6/60 segments. If any AA/6/60 segment(s) is/are present then this/these will not include the AA/6/60 HA segment and usually will not include the AA/6/60 NA segment. Thus preferred viruses are those in which at least one of segments NP, M, NS, PA, PB1 and/or PB2 is not derived from AA/6/60. More preferably, at least one of segments NP, M, PA, PB1 and/or PB2 is not derived from AA/6/60.

Preferred influenza B viruses include fewer than 6 *(i.e.* 0, 1, 2, 3, 4 or 5) viral segments from an AA/1/66 influenza virus (B/Ann Arbor/1/66). Preferably they include no AA/1/66 segments. If any AA/1/66 segment(s) is/are present then this/these will not include the AA/1/66 HA segment and usually will not include the AA/1/66 NA segment. Thus preferred viruses are those in which at least one of segments NP, M, NS, PA, PB1 and/or PB2 is not derived from AA/1/66. More preferably, at least one of segments NP, M, PA, PB1 and/or PB2 is not derived from AA/1/66.

Preferred influenza viruses of the invention (including seed viruses, viruses isolated from patient samples using MDCK cells, reassortant viruses, *etc.*) include hemagglutinin with a binding preference for oligosaccharides with a Sia(α2,6)Gal terminal disaccharide compared to oligosaccharides with a Sia(α2,3)Gal terminal disaccharide. This binding preference is discussed in more detail above.

Preferred influenza viruses of the invention (including seed viruses, viruses isolated from patient samples using MDCK cells, reassortant viruses, *etc*.) include glycoproteins (including hemagglutinin) with a different glycosylation pattern from egg-derived viruses. Thus the glycoproteins will comprise glycoforms that are not seen in viruses grown in chicken eggs e.g. they may have non-avian sugar linkages, including mammalian sugar linkages.

### Cell lines

The invention involves the use of cell lines that support influenza virus replication, and avoids the use of eggs. The cell line will typically be of mammalian origin. Suitable mammalian cells of origin include, but are not limited to, hamster, cattle, primate (including humans and monkeys) and dog cells, although the use of primate cells is not preferred. Various cell types may be used, such as kidney cells, fibroblasts, retinal cells, lung cells, *etc.* Examples of suitable hamster cells are the cell lines having the names BHK21 or HKCC. Suitable monkey cells are *e.g.* African green monkey cells, such as kidney cells as in the Vero cell line [21-23]. Suitable dog cells are e.g. kidney cells, as in the CLDK and MDCK cell lines.

Thus suitable cell lines include, but are not limited to: MDCK; CHO; CLDK; HKCC; 293T; BHK; Vero; MRC-5; PER.C6 [24]; FRhL2; WI-38; *etc.* Suitable cell lines are widely available *e.g.* from the American Type Cell Culture (ATCC) collection [25], from the Coriell Cell Repositories [26], or from the European Collection of Cell Cultures (ECACC). For example, the ATCC supplies various different Vero cells under catalog numbers CCL-81, CCL-81.2, CRL-1586 and CRL-1587, and it supplies MDCK cells under catalog number CCL-34. PER.C6 is available from the ECACC under deposit number 96022940. Any of these cell types can be used for growth, reassortment and/or passaging according to the invention.

The most preferred cell lines are those with mammalian-type glycosylation. As a less-preferred alternative to mammalian cell lines, virus can be grown on avian cell lines [*e.g.* refs. 27-29], including cell lines derived from ducks *(e.g.* duck retina) or hens *e.g.* chicken embryo fibroblasts (CEF), *etc.,* but the use of mammalian cells means that vaccines can be free from avian DNA and egg proteins (such as ovalbumin and ovomucoid), thereby reducing allergenicity.

The most preferred cell lines for growing influenza viruses are MDCK cell lines [30-33], derived from Madin Darby canine kidney. The original MDCK cell line is available from the ATCC as CCL-34, but derivatives of this cell line may also be used. For instance, reference 30 discloses a MDCK cell line that was adapted for growth in suspension culture ('MDCK 33016' or '33016-PF', deposited as DSM ACC 2219; see also refs. 34 & 35). Similarly, reference 36 discloses a MDCK-derived cell line that grows in suspension in serum-free culture ('B-702', deposited as FERM BP-7449). Reference 37 discloses non-tumorigenic MDCK cells, including 'MDCK-S' (ATCC PTA-6500), 'MDCK-SF101' (ATCC PTA-6501), 'MDCK-SF102' (ATCC PTA-6502) and MDCK-SF103' (ATCC PTA-6503). Reference 38 discloses MDCK cell lines with high susceptibility to infection, including 'MDCK.5F1' cells (ATCC CRL-12042) Any of these MDCK cell lines can be used with the invention.

Virus may be grown on cells in adherent culture or in suspension. Microcarrier cultures can also be used. In some embodiments, the cells may thus be adapted for growth in suspension.

Cell lines are preferably grown in serum-free culture media and/or protein free media. In the context of the present invention a medium is referred to as a serum-free medium when it has no additives from serum of human or animal origin. The cells growing in such cultures naturally contain proteins themselves, but a protein-free medium is understood to mean one in which multiplication of the cells occurs with exclusion of (without addition to the culture medium of) proteins, growth factors, other protein additives and non-serum proteins, but can optionally include (in the culture medium) proteins such as trypsin or other proteases that may be necessary for viral growth.

Cell lines supporting influenza virus replication are preferably grown below 37°C [39] *(e.g.* 30-36°C, or at about 30°C, 31°C, 32°C, 33°C, 34°C, 35°C, 36°C) during viral replication. For instance, in the sixth aspect, MDCK cells may be grown (before, during or after the isolation step) at these temperatures, particularly during viral replication.

Methods for propagating influenza virus in cultured cells *(e.g.* for growing influenza virus in cultured MDCK cells according to the sixth aspect) generally includes the steps of inoculating a culture of cells with an inoculum of the strain to be grown, cultivating the infected cells for a desired time period for virus propagation, such as for example as determined by virus titer or antigen expression (*e.g*. between 24 and 168 hours after inoculation) and collecting the propagated virus. The cultured cells are inoculated with a virus (measured by PFU or TCID₅₀) to cell ratio of 1:500 to 1:1, preferably 1:100 to 1:5, more preferably 1:50 to 1:10. The virus is added to a suspension of the cells or is applied to a monolayer of the cells, and the virus is absorbed on the cells for at least 60 minutes but usually less than 300 minutes, preferably between 90 and 240 minutes at 25°C to 40°C, preferably 28°C to 37°C. The infected cell culture (*e.g*. monolayers) may be removed either by freeze-thawing or by enzymatic action to increase the viral content of the harvested culture supernatants. The harvested fluids are then either inactivated or stored frozen. Cultured cells may be infected at a multiplicity of infection ("m.o.i.") of about 0.0001 to 10, preferably 0.002 to 5, more preferably to 0.001 to 2. Still more preferably, the cells are infected at a m.o.i of about 0.01. Infected cells may be harvested 30 to 60 hours post infection. Preferably, the cells are harvested 34 to 48 hours post infection. Still more preferably, the cells are harvested 38 to 40 hours post infection. Proteases (typically trypsin) are generally added during cell culture to allow viral release, and the proteases can be added at any suitable stage during the culture e.g. before inoculation, at the same time as inoculation, or after inoculation [39].

In preferred embodiments, particularly with MDCK cells, a cell line is not passaged from a master working cell bank beyond 40 population-doubling levels.

The viral inoculum and the viral culture are preferably free from *(i.e.* will have been tested for and given a negative result for contamination by) herpes simplex virus, respiratory syncytial virus, parainfluenza virus 3, SARS coronavirus, adenovirus, rhinovirus, reoviruses, polyomaviruses, birnaviruses, circoviruses, and/or parvoviruses [40]. Similarly, preferred MDCK cell lines used with the sixth aspect are free from (*i.e.* will have been tested for and given a negative result for infection by) herpes simplex viruses, respiratory syncytial viruses, parainfluenza virus 3, SARS coronavirus, adenoviruses, rhinoviruses, reoviruses, polyomaviruses, birnaviruses, circoviruses, and/or parvoviruses. Absence of herpes simplex viruses is particularly preferred.

A MDCK cell line used with the invention preferably contains no marker for G418 resistance (*cf.* reference 16). Thus the cell line may be sensitive to G418 treatment.

The cell line used with the invention preferably contains no exogenous plasmids (*cf.* reference 16), except for any that may be required for reverse genetics techniques.

### General

The term "comprising" encompasses "including" as well as "consisting" *e.g.* a composition "comprising" X may consist exclusively of X or may include something additional e.g. X + Y.

The word "substantially" does not exclude "completely" *e.g.* a composition which is "substantially free" from Y may be completely free from Y. Where necessary, the word "substantially" may be omitted from the definition of the invention.

The term "about" in relation to a numerical value x means, for example, x±10%.

Unless specifically stated, a process comprising a step of mixing two or more components does not require any specific order of mixing. Thus components can be mixed in any order. Where there are three components then two components can be combined with each other, and then the combination may be combined with the third component, *etc.*

Where animal (and particularly bovine) materials are used in the culture of cells, they should be obtained from sources that are free from transmissible spongiform encaphalopathies (TSEs), and in particular free from bovine spongiform encephalopathy (BSE). Overall, it is preferred to culture cells in the total absence of animal-derived materials.

Where a compound is administered to the body as part of a composition then that compound may alternatively be replaced by a suitable prodrug.

### BRIEF DESCRIPTION OF THE DRAWINGS

Figure 1 shows the scheme of isolation of influenza virus from clinical specimens.
Figure 2 compares HA titers of 9 viral samples isolated in MDCK-33016 cells. In each sample, the left-hand bar is at passage 2 and the right-hand bar is at passage 5.
Figure 3 compared HA titers of 10 samples of influenza viruses grown in three different MDCK cell types. For each sample, the three bars are: left, 33016 in suspension; middle, adherent 33016; and right, CCL-34 MDCK cells.
Figure 4 shows the binding of three viruses to SNA or MAA lectins. Figure 4A shows binding of an original isolate, 4B shows binding after growth in MDCK 33016 cells, and 4C shows binding after growth in eggs.

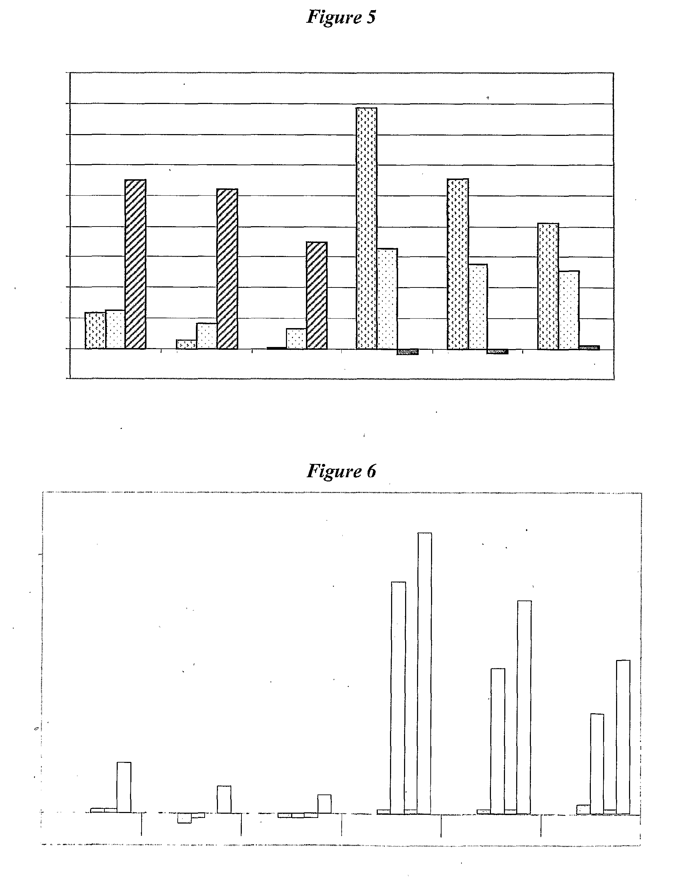

Figures 5 and 6 show the binding of viruses to 3-SL or 6-SLN. In both figures there are six groups of columns: the left-most three show binding to 3-SL at different concentrations (1µM, 0.5µM, 0.25µM) and the right-most three show binding to 6-SLN at different concentrations (0.25µM, 0.125µM, 0.0625µM). Within each of the six groups, each column shows a different virus. In Figure 5, the three columns are, from left to right: (i) a cell-isolated virus; (ii) an egg-isolated virus; and (iii) an avian virus. In Figure 6, the four columns are, from left to right: (i) virus after two passages in eggs; (ii) virus after two passages in MDCK; (iii) virus after five passages in eggs; (iv) virus after five passages in MDCK.

### MODES FOR CARRYING OUT THE INVENTION

### Virus isolation from patient samples

Clinical specimens (nasal or throat swabs) containing influenza A and/or B virus subtypes were obtained from children and adults during the 2006-2007 northern hemisphere influenza season. The susceptibility and reliability of the MDCK 33016 cell-line (DSM ACC 2219) grown in serum-free suspension culture was compared with the established MDCK CCL 34 cell-line (ATCC) and with chicken eggs, by determination of hemagglutinin (HA) titers, polymerase chain reaction (PCR), and virus titration. 248 influenza positive samples were identified by diagnostic polymerase chain reaction (PCR). Susceptibility and reliability of influenza virus replication and isolation was assessed in the MDCK 33016 cell-line and in chicken eggs by: (i) hemagglutinin (HA) titers; (ii) real-time polymerase chain reaction (PCR) for viral load measurement; and (iii) virus titration. Replication accuracy in the cells was assessed by sequencing the HA gene in the original clinical specimens and also in isolates from the second passage in MDCK cells and the chicken eggs. Virus titers obtained from isolates grown in suspension MDCK 33016 cells were compared to those from MDCK 33016 cells adhered on plates.

Results indicated that the isolation capacity of the MDCK 33016 suspension cell-line is superior to the established MDCK CCL 34 cell-line, and much greater than that of chicken eggs. After passage of virus samples in MDCK 33016 cells, amino acid substitutions were identified in no isolates. In contrast, nearly all egg-passaged viruses contained one or more amino acid substitutions, predominately in the HA1 gene. Mutations in the antibody binding site of the HA gene, observed following passage in eggs, may result in modifications to the antigenicity of the influenza virus.

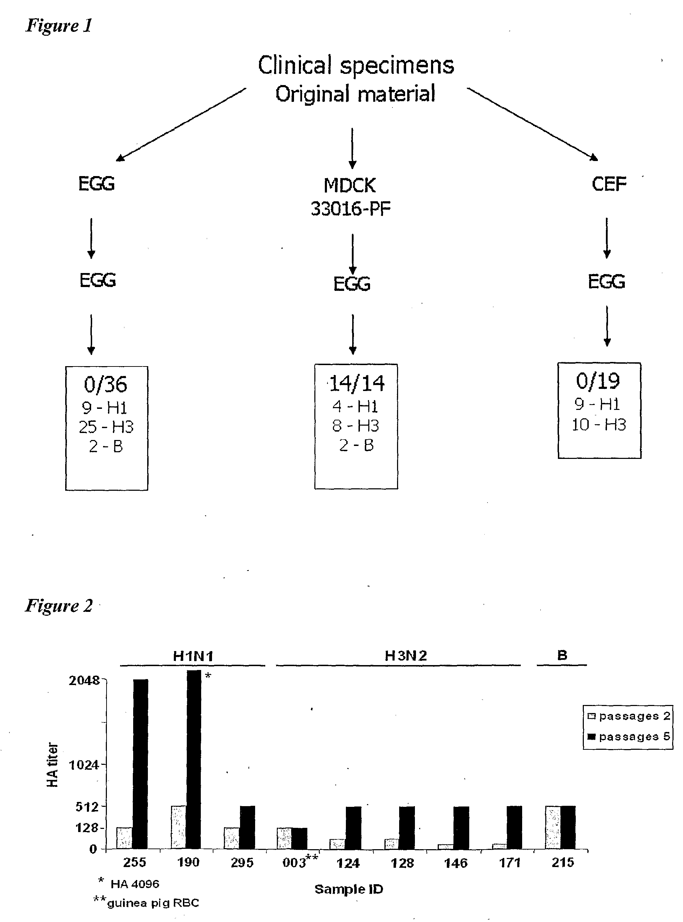

55% of clinical samples obtained from patients with acute respiratory disease, were identified as influenza positive, with the following viral types: 79% A/H3N2; 12.5% A/H1N1; 1.6% B, 0.4% H3/B and 6.5% untypeable. Viral isolation from clinical specimens was possible using MDCK 33016 cells (Figure 1). In contrast, of the viruses injected into eggs from clinical specimens, none was successfully isolated. Similar negative results were achieved with freshly prepared chicken embryo fibroblasts (CEF). Isolation and establishment of influenza virus in eggs could only be achieved using supernatants of MDCK 33016 cultures with a positive HA titer.

The first harvest from each cell was further inoculated into eggs, for reference purposes. The number of successful virus isolations, using each approach, is shown in the boxes in Figure 1, with the number of different viral types injected also shown. All three virus subtypes isolated from the MDCK 33016 cells, gained reasonable HA (>32) and virus titer (>1x10⁶) after the second passage in eggs, with both titers increasing with further passages (Figure 2).

MDCK 33016 cells growing in suspension were superior to the adherent cell line (CCL-34) for isolation of influenza virus from clinical swabs for all three subtypes. The suspension cell line showed a higher sensitivity for positive influenza swab material as demonstrated by the recovery rate (Table 1). HA sequences were compared between different passages in MDCK 33016 cells and eggs to the original isolate (Table 2), and no mutations were found for influenza A strains isolated in MDCK 33016 cells even after 5 passages, whereas influenza A strains isolated in eggs showed mutations in the antibody binding site of the HA protein after 2 passages. No mutations were found for influenza B strains isolated in MDCK 33016 cells or eggs.

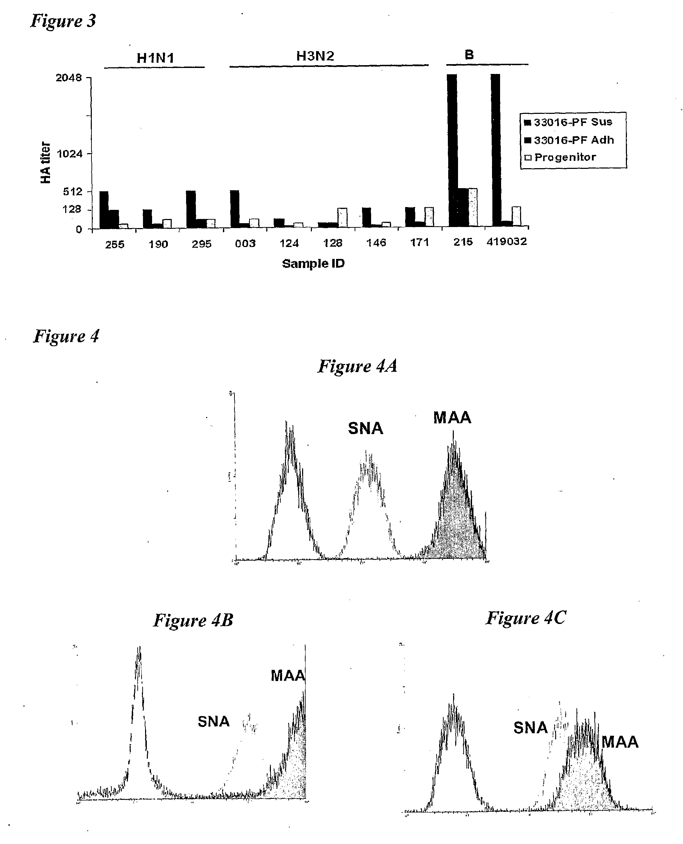

Higher virus yields, of at least one log level, were found following replication of isolates in suspension MDCK 33016 cells compared with adhered MDCK 33016 cells (Figure 3).

Thus the MDCK 33016 suspension cell-line is an ideal system for the isolation and replication of wild-type influenza strains, as it offers a greater isolation capacity compared with chicken eggs. Furthermore, due to the high replication accuracy, the use of cell-based isolates for the production of a human influenza vaccine may lead to a more authentic vaccine. Improved match between the circulating wild-type strains and those contained in the vaccine should offer greater protection against influenza for the vaccinee.

In conclusion: (a) all virus strains were successfully isolated in MDCK 33016 cells compared to eggs; (b) virus strains isolated from MDCK 33016 cells could be propagated successfully in eggs; (c) the recovery rate of all three influenza virus subtypes is superior in MDCK 33016 cells grown in suspension, when compared to the adherent cells; and (d) substitutions of the HA gene, when compared to the original material, were not present in any of the isolates grown in MDCK 33016 cells but were present after the second passage in eggs. Thus the MDCK 33016 suspension cell-line is a very suitable substrate for isolation and propagation of human influenza virus subtypes as it is highly reliable for passaging wild type influenza virus from clinical isolates and is preserves an authentic character of the wild type virus.

### Receptor binding

The receptor preferences of original isolated viruses, of egg-grown viruses and of MDCK-grown viruses were investigated. Studies used lectins with 2,3-sialyl linkages (MAA) or 2,6-sialyl linkages (SNA), or 2,3-sialyllactose (3-SL, an analog of the egg receptor) and 2,6-sialyl-N-acetyllactosamine (6-SLN, an analog of the human receptor) sialylglycopolymers [193].

Figure 4 shows the results of a representative study. The labelled peaks show binding to the SNA or MAA lectins. The original virus (4A) and the virus grown on MDCK 33016 (4B) have distinct peaks for SNA & MAA, whereas the SNA & MAA peaks substantially overlap for egg-grown virus (4C).

Binding specificity was also examined in further experiments using 3-SL and 6-SLN. An example result is shown in Figure 5. Binding on the left of the graph indicates an avian receptor preference, whereas binding on the right indicates a human receptor preference. As visible in Figure 5, the cell-isolated virus strongly favours human receptors.

Figure 6 shows data using a Stuttgart isolate (A/H1N1) after 2 or 5 passages in eggs or in MDCK 33016 grown in suspension. The MDCK-passaged viruses show a strong preference for 6-SLN.

In conclusion, all MDCK-grown clinical human A and B viruses bind to 6-SLN rather than to 3-SL, except that some isolates were found that bind to neither in this assay. Unlike the original clinical isolates, egg-adapted viruses bind either to 3-SL or to neither 3-SL nor 6-SLN.

### Changes due to growth in eggs

Various strains of influenza A and B virus were isolated in MDCK cells and then passaged up to five times through one of the following substrates: eggs; MDCK cells CCL-34; MDCK cells 33016; Vero cells; or HEK 293-T cells. The HA gene of the viruses were sequenced after each passage and HA titres were measured.

Although the HA sequence for some strains (*e.g*. A/H1N1/Bayern/7/95) was stable during passage through eggs and through MDCK 33016, for others it was not. For instance, the HA sequence of A/H1N1/Nordrhein Westfalen/1/05 acquired a mutation D203N at antibody binding site D after 2 passages through eggs, and after 2 more passages it additionally acquired a R329K. In contrast, the sequence was unaltered in viruses passaged in parallel through MDCK 33016.

For this A/H1N1/NRW/1/05 strain, growth was not seen when cultured with Vero cells The other four substrates could support its growth, but the HA titres varied. For instance, titres of 32-256 were seen in eggs, but 293-T cells gave lower titres (16-32) and MDCK 33016 gave higher titres (32-512).

It will be understood that the invention has been described by way of example only and modifications may be made whilst remaining within the scope of the invention.

**TABLE 1: Recovery rate after the first passage of influenza positive samples in MDCK 33016 and ATCC (CCL-34) cell lines**

| | | **Recovery rate n (%) according to viral strain** | | | |
|---|---|---|---|---|---|
| | **Total n=248* (%)** | **A/H1N1** | **A/H3N2** | **B** | **Untypeable** |
| | | **(n=31)** | **(n=196)** | **(n=4)** | **(n=16)** |
| 33016 | 178(72) | 26 (83.9) | 150 (76.5) | 4 (100) | 9 (56.3) |
| CCL-34 | 156(63) | 23 (74.2) | 135 (68.9) | 2 (50) | 4 (25.0) |

| | | | | | |
|---|---|---|---|---|---|
| * 1 double infected (H3/B) which could be isolated in both MDCK cell lines | | | | | |

**TABLE 2: Comparison of hemagglutinin sequences after 2 or 5 passages in MDCK 33016-PF cells or eggs to the original isolate**

| **Isolate (serotype)** | **Passage (host)** | **Comparison to the original material (nucleotide)** | **Comparison to the original material (amino acid)** |
|---|---|---|---|
| 295 (H1N1) | P2 (MDCK) | 0* | 0* |
| | P2 (egg) | 1* | D203N* |
| 124 (H3N2) | P2 (MDCK) | 0 | 0 |
| | P5 (MDCK) | 0 | 0 |
| | P2 (egg) | 1 | L210P |
| 128 (H3N2) | P2 (MDCK) | 0 | 0 |
| | P5 (MDCK) | 0 | 0 |
| | P2 (egg) | 3 | L210P |
| 146 (H3N2) | P2 (MDCK) | 0 | 0 |
| | P5 (MDCK) | 0 | 0 |
| | P2 (egg) | 1 | L210P |
| 171 (H3N2) | P2 (MDCK) | 0 | 0 |
| | P5 (MDCK) | 0 | 0 |
| | P2 (egg) | 1 | H199L |
| 215 (B) | P5 (MDCK) | 0** | 0** |
| | P2 (egg) | 0** | 0** |
| 419032(B) | P2 (MDCK) | 0 | 0 |
| | P2 (egg) | 0 | 0 |

| | | | |
|---|---|---|---|
| 0 = no mutation detectable * for original isolate only the HA1 sequence was available ** comparison to the P2 (MDCK 33016) isolate | | | |

### REFERENCES

[1] Gerdil (2003) Vaccine 21:1776-9.
[2] Palese (2006) Emerging Infectious Diseases 12:61-65.
[3] de Oña et al. (1995) J Clin Microbiol 33:1948-49.
[4] Monto et al. (1981) J Clin Microbiol13(1): 233-235.
[5] Govorkova et al. (1995) J Infect Dis. 172(1):250-3.
[6] Tobita et al. (1975) Med Microbiol Immunol (Berl). 162(1):9-14 and 23-27.
[7] Ollier et al. (2004) J Clin Microbiol 42(12):5861-5.
[8] Schepetiuk & Kok (1993) J Virol Methods 42(2-3):241-50.
[9] WHO (2003) Weekly epidemiological record 78:73-80
[10] US patent 6,344,354. See also WO97/38094.
[11] US patent 5,162,112
[12] Medema et al. (2004) Virus Res. 103(1-2):9-15.
[13] Robertson et al. (1995) Vaccine 13:1583-8.
[14] Merten et al. (1996) Advances in Experimental Biology 397:141-151.
[15] Govorkova et al. (1996) J. Virol., 70:5519-24.
[16] Mastrosovich et al. (2003) J Virol 77:8418-25.
[17] Gambaryan & Matrosovich (1992) J Virol Methods 39(1-2):111-23.
[18] Mastrosovich et al. (1999) J Virol 73: 1146-55.
[19] Stevens et al. (2006) J Mol Biol 355:1143-55.
[20] Couceiro & Baum (1994) Mem Inst Oswaldo Cruz 89(4):587-91.
[21] Kistner et al. (1998) Vaccine 16:960-8.
[22] Kistner et al. (1999) Dev Biol Stand 98:101-110.
[23] Bruhl et al. (2000) Vaccine 19:1149-58.
[24] Pau et al. (2001) Vaccine 19:2716-21.
*[25] http:*//*www.atcc.org*/
*[26] http:*//*locus.umdnj.edu*/
[27] WO03/076601.
[28] WO2005/042728.
[29] WO03/043415.
[30] WO97/37000.
[31] Brands et al. (1999) Dev Biol Stand 98:93-100.
[32] Halperin et al. (2002) Vaccine 20:1240-7.
[33] Tree et al. (2001) Vaccine 19:3444-50.
[34] WO03/023021
[35] WO03/023025
[36] EP-A-1260581 (WO01/64846).
[37] WO2006/071563.
[38] WO2005/113758.
[39] WO97/37001.
[40] WO2006/027698.
[41] Hoffmann et al. (2002) Vaccine 20:3165-3170.
[42] Subbarao et al. (2003) Virology 305:192-200.
[43] Liu et al. (2003) Virology 314:580-590*.*
[44] Ozaki et al. (2004) J. Virol. 78:1851-1857.
[45] Webby et al. (2004) Lancet 363:1099-1103.
[46] WO00/60050.
[47] WO01/04333.
[48] US patent 6649372.
[49] Neumann et al. (2005) Proc Natl Acad Sci USA 102:16825-9.
[50] WO2007/002008.
[51] WO2006/067211.
[52] WO01/83794.
[53] Hoffmann et al. (2000) Virology 267(2):310-7.
*[54]* Vaccines. (eds. Plotkin & Orenstein). 4th edition, 2004, ISBN: 0-7216-9688-0.
[55] WO02/28422.
[56] WO02/067983.
[57] WO02/074336.
[58] WO01/21151.
[59] WO02/097072.
[60] WO2005/113756.
[61] Huckriede et al. (2003) Methods Enzymol 373:74-91.
[62] Treanor et al. (1996) J Infect Dis 173:1467-70.
[63] Keitel et al. (1996) Clin Diagn Lab Immunol 3:507-10.
[64] World Health Organisation (2005) Emerging Infectious Diseases 11(10):1515-21.
[65] Herlocher et al. (2004) J Infect Dis 190(9):1627-30.
[66] Le et al. (2005) Nature 437(7062):1108.
[67] EP-B-0870508.
[68] US 5948410.
[69] WO2007/052163.
[70] Lundblad (2001) Biotechnology and Applied Biochemistry 34:195-197.
[71] Guidance for Industry: Bioanalytical Method Validation. U.S. Department of Health and Human Services Food and Drug Administration Center for Drug Evaluation and Research (CDER) Center for Veterinary Medicine (CVM). May 2001.
[72] Ji et al. (2002) Biotechniques. 32:1162-7.
[73] Briggs (1991) J Parenter Sci Technol. 45:7-12.
[74] Lahijani et al. (1998) Hum Gene Ther. 9:1173-80.
[75] Lokteff et al. (2001) Biologicals. 29:123-32.
[76] Gennaro (2000) Remington: The Science and Practice ofPharmacy. 20th edition, ISBN: 0683306472.
[77] Banzhoff (2000) Immunology Letters 71:91-96.
[78] Nony et al. (2001) Vaccine 27:3645-51.
[79] US patent 6,372,223.
[80] WO00/15251.
[81] WO01/22992.
[82] Hehme et al. (2004) Virus Res. 103(1-2):163-71.
[83] US patent 6355271.
[84] WO00/23105.
[85] US 5,057,540.
[86] WO96/33739.
[87] EP-A-0109942.
[88] WO96/11711.
[89] WO00/07621.
[90] Barr et al. (1998) Advanced Drug Delivery Reviews 32:247-271.
[91] Sjolanderet et al. (1998) Advanced Drug Delivery Reviews 32:321-338.
[92] Pizza et al. (2000) Int J Med Microbiol 290:455-461.
[93] WO95/17211.
[94] WO98/42375.
[95] Singh et al] (2001) J Cont Release 70:267-276.
[96] WO99/27960.
[97] US 6,090,406
[98] US 5,916,588
[99] EP-A-0626169.
[100] WO99/52549.
[101] WO01/21207.
[102] WO01/21152.
[103] WO02/072012.
[104] Signorelli & Hadden (2003) Int Immunopharmacol 3(8):1177-86.
[105] WO2004/064715.
[106] Cooper (1995) Pharm Biotechnol 6:559-80.
[107] Dyakonova et al. (2004) Int Immunopharmacol 4(13):1615-23.
[108] FR-2859633.
[109] WO90/14837.
[110] Podda & Del Giudice (2003) Expert Rev Vaccines 2:197-203.
[111] Podda (2001) Vaccine 19: 2673-2680.
[112] Vaccine Design: The Subunit and Adjuvant Approach (eds. Powell & Newman) Plenum Press 1995 (ISBN 0-306-44867-X).
[113] Vaccine Adjuvants: Preparation Methods and Research Protocols (Volume 42 of Methods in Molecular Medicine series). ISBN: 1-59259-083-7. Ed. O'Hagan.
[114] Allison & Byars (1992) Res Immunol 143:519-25.
[115] Hariharan et al. (1995) Cancer Res 55:3486-9.
[116] US-2007/014805.
[117] Suli et al. (2004) Vaccine 22(25-26):3464-9.
[118] WO95/11700.
[119] US patent 6,080,725.
[120] WO2006/113373.
[121] WO2005/097181.
[122] Han et al. (2005) Impact of Vitamin E on Immune Function and Infectious Diseases in the Aged at Nutrition, Immune functions and Health EuroConference, Paris, 9-10 June 2005.
[123] US-6630161.
[124] Hayden et al. (1998) J Clin Invest 101(3):643-9.
[125] Tassignon et al. (2005) J Immunol Meth 305:188-98.
[126] Myers et al. (1990) pages 145-156 of Cellular and molecular aspects of endotoxin reactions*.*
[127] Ulrich (2000) Chapter 16 (pages 273-282) of reference 113.
[128] Johnson et al. (1999) J Med Chem 42:4640-9.
[129] Baldrick et al. (2002) Regulatory Toxicol Pharmacol 35:398-413.
[130] US 4,680,338.
[131] US 4,988,815.
[132] WO92/15582.
[133] Stanley (2002) Clin Exp Dermatol 27:571-577.
[134] Wu et al. (2004) Antiviral Res. 64(2):79-83.
[135] Vasilakos et al. (2000) Cell Immunol. 204(1):64-74.
[136] US patents 4689338, 4929624, 5238944, 5266575, 5268376, 5346905, 5352784, 5389640, 5395937, 5482936, 5494916, 5525612, 6083505, 6440992, 6627640, 6656938, 6660735, 6660747, 6664260, 6664264, 6664265, 6667312, 6670372, 6677347, 6677348, 6677349, 6683088, 6703402, 6743920, 6800624, 6809203, 6888000 and 6924293.
[137] Jones (2003) Curr Opin Investig Drugs 4:214-218.
[138] WO2004/060308.
[139] WO2004/064759.
[140] US 6,924,271.
[141] US2005/0070556.
[142] US 5,658,731.
[143] US patent 5,011,828.
[144] WO2004/87153.
[145] US 6,605,617.
[146] WO02/18383.
[147] WO2004/018455.
[148] WO03/082272.
[149] WO2006/002422.
[150] Johnson et al. (1999) Bioorg Med Chem Lett 9:2273-2278.
[151] Evans et al. (2003) Expert Rev Vaccines 2:219-229.
[152] Andrianov et al. (1998) Biomaterials 19:109-115.
[153] Payne et al. (1998) Adv Drug Delivery Review 31:185-196.
[154] Thompson et al. (2003) Methods in Molecular Medicine 94:255-266.
[155] Kandimalla et al. (2003) Nucleic Acids Research 31:2393-2400.
[156] WO02/26757.
[157] WO99/62923.
[158] Krieg (2003) Nature Medicine 9:831-835.
[159] McCluskie et al. (2002) FEMS Immunology and Medical Microbiology 32:179-185.
[160] WO98/40100.
[161] US patent 6,207,646.
[162] US patent 6,239,116.
[163] US patent 6,429,199.
[164] Kandimalla et al. (2003) Biochemical Society Transactions 31 (part 3):654-658.
[165] Blackwell et al. (2003) J Immunol 170:4061-4068.
[166] Krieg (2002) Trends Immunol 23:64-65.
[167] WO01/95935.
[168] Kandimalla et al. (2003) BBRC 306:948-953.
[169] Bhagat et al. (2003) BBRC 300:853-861.
[170] WO03/035836.
[171] WO01/22972.
[172] Thompson et al. (2005) J Leukoc Biol 78: 'The low-toxicity versions of LPS, MPL® adjuvant and RC529, are efficient adjuvants for CD4+ T cells'.
[173] UK patent application GB-A-2220211.
[174] WO 94/21292.
[175] WO94/00153.
[176] WO95/17210.
[177] WO96/26741.
[178] WO93/19780.
[179] WO03/011223.
[180] Meraldi et al. (2003) Vaccine 21:2485-2491.
[181] Pajak et al. (2003) Vaccine 21:836-842.
[182] US-6586409.
[183] Wong et al. (2003) J Clin Pharmacol 43(7):735-42.
[184] US2005/0215517.
[185] Potter & Oxford (1979) BrMed Bull 35: 69-75.
[186] Greenbaum et al. (2004) Vaccine 22:2566-77.
[187] Zurbriggen et al. (2003) Expert Rev Vaccines 2:295-304.
[188] Piascik (2003) J am Pharm Assoc (Wash DC). 43:728-30.
[189] Mann et al. (2004) Vaccine 22:2425-9.
[190] Halperin et al. (1979) Am J Public Health 69:1247-50.
[191] Herbert et al. (1979) J Infect Dis 140:234-8.
[192] Chen et al. (2003) Vaccine 21:2830-6.
[193] Gambaryan et al. (1997) Virology 232:345-50.

## Claims

1. A process for preparing an antiserum from a non-human animal, comprising steps of:
(i) administering to the non-human animal a purified influenza virus hemagglutinin; and then
(ii) recovering from the non-human animal serum containing antibodies that recognise the hemagglutinin, **characterised in that** the hemagglutinin used in step (i) is from a virus grown in a mammalian cell line.

2. A process for preparing an antiserum from a non-human animal, comprising steps of:
(i) growing influenza virus in a mammalian cell line;
(ii) purifying hemagglutinin antigen from virus grown in step (i);
(iii) administering the purified hemagglutinin from step (ii) to the non-human animal; and then
(iv) recovering from the non-human animal serum containing antibodies that recognise the hemagglutinin.

3. The process of claim 1 or claim 2, wherein the non-human animal is a sheep.

4. The process of any preceding claim, wherein the hemagglutinin is free from egg-derived materials.

5. The process of any preceding claim, including the further step of mixing the antiserum with a gel suitable for a SRID assay.

6. The process of claim 1 wherein the hemagglutinin used in step (i) is from a virus that has never been grown in eggs.

7. The process of claim 1 wherein the hemagglutinin used to raise the antiserum is glycosylated with glycans obtainable from growth in a mammalian cell line.

8. A process for preparing a kit, comprising the steps of (i) making an antiserum according to one of claims 1 to 4; (ii) making an antigen reference material by a process comprising the steps of (a) growing influenza virus in a cell line; (b) purifying viruses grown in step (a); and (c) inactivating the virus, **characterised in that** the influenza virus used in step (a) has never been grown in eggs; and (iii) combining the products of steps (i) and (ii) into a kit.

9. The process of any one of claims 1 to 8 wherein the cell line is a MDCK cell line.

10. The process of claim 9 wherein the MDCK cell line is MDCK33016.

## Patentansprüche

1. Verfahren zur Herstellung eines Antiserums aus einem nichtmenschlichen Tier, umfassend die folgenden Schritte:
(i) Verabreichen eines aufgereinigten Influenzavirushämagglutinins an das nichtmenschliche Tier; und danach
(ii) Gewinnen von Serum, enthaltend Antikörper, die das Hämagglutinin erkennen, aus dem nichtmenschlichen Tier, **dadurch gekennzeichnet, dass** das in Schritt (i) verwendete Hämagglutinin von einem Virus, das in einer Säugetierzelllinie gezüchtet wurde, stammt.

2. Verfahren zur Herstellung eines Antiserums aus einem nichtmenschlichen Tier, umfassend die folgenden Schritte:
(i) Züchten von Influenzavirus in einer Säugetierzelllinie;
(ii) Aufreinigen von Hämagglutininantigen aus dem in Schritt (i) gezüchteten Virus; und
(iii) Verabreichen des aufgereinigten Hämagglutinins aus Schritt (ii) an das nichtmenschliche Tier; und dann
(iv) Gewinnen von Serum, das Antikörper enthält, die das Hämagglutinin erkennen, aus dem nichtmenschlichen Tier.

3. Verfahren nach Anspruch 1 oder Anspruch 2, wobei es sich bei dem nichtmenschlichen Tier um ein Schaf handelt.

4. Verfahren nach einem vorhergehenden Anspruch, wobei das Hämagglutinin frei von aus Eiern stammenden Substanzen ist.

5. Verfahren nach einem vorhergehenden Anspruch, das weiterhin den folgenden Schritt umfasst: Mischen des Antiserums mit einem Gel, das sich für einen SRID-Assay eignet.

6. Verfahren nach Anspruch 1, wobei das in Schritt (i) eingesetzte Hämagglutinin von einem Virus stammt, das niemals in Eiern gezüchtet worden ist.

7. Verfahren nach Anspruch 1, wobei das Hämagglutinin, das zur Gewinnung des Antiserums verwendet wurde, mit Glykanen glykosyliert wird, die durch Wachstum in einer Säugetierzelllinie erhältlich sind.

8. Verfahren zur Herstellung eines Kits, umfassend die folgenden Schritte: (i) Herstellen eines Antiserums nach einem der Ansprüche 1 bis 4; (ii) Herstellen eines antigenen Referenzmaterials durch ein Verfahren, umfassend die Schritte: (a) Züchten von Influenzavirus in einer Zelllinie; (b) Aufreinigen der in Schritt (a) gezüchteten Viren; und (c) Inaktivieren des Virus, **dadurch gekennzeichnet, dass** das in Schritt (a) verwendete Influenzavirus niemals in Eiern gezüchtet worden ist; und (iii) Vereinigen der Produkte der Schritte (i) und (ii) zu einem Kit.

9. Verfahren nach einem der Ansprüche 1 bis 8, wobei es sich bei der Zelllinie um eine MDCK-Zelllinie handelt.

10. Verfahren nach Anspruch 9, wobei es sich bei der MDCK-Zelllinie um MDCK33016 handelt.

## Revendications

1. Procédé de préparation d'un antisérum à partir d'un animal non humain, comprenant les étapes de :
(i) administration à l'animal non humain d'une hémagglutinine de virus de la grippe purifiée ; et ensuite
(ii) récupération à partir de l'animal non humain de sérum contenant des anticorps qui reconnaissent l'hémagglutinine, **caractérisé en ce que** l'hémagglutinine utilisée dans l'étape (i) provient d'un virus cultivé dans une lignée cellulaire de mammifère.

2. Procédé de préparation d'un antisérum à partir d'un animal non humain, comprenant les étapes de :
(i) culture de virus de la grippe dans une lignée cellulaire de mammifère ;
(ii) purification d'antigène d'hémagglutinine à partir du virus cultivé dans l'étape (i) ;
(iii) administration de l'hémagglutinine purifiée de l'étape (ii) à l'animal non humain ; et ensuite
(iv) récupération à partir de l'animal non humain de sérum contenant des anticorps qui reconnaissent l'hémagglutinine.

3. Procédé de la revendication 1 ou la revendication 2, dans lequel l'animal non humain est un mouton.

4. Procédé de l'une quelconque des revendications précédentes, dans lequel l'hémagglutinine est exempte de matériaux dérivés d'oeuf.

5. Procédé de l'une quelconque des revendications précédentes, comprenant l'étape supplémentaire de mélange de l'antisérum avec un gel adapté pour un essai SRID.

6. Procédé de la revendication 1 dans lequel l'hémagglutinine utilisée dans l'étape (i) provient d'un virus qui n'a jamais été cultivé dans des oeufs.

7. Procédé de la revendication 1 dans lequel l'hémagglutinine utilisée pour induire l'antisérum est glycosylée avec des glycanes pouvant être obtenus à partir de la culture dans une lignée cellulaire de mammifère.

8. Procédé de préparation d'un kit, comprenant les étapes de (i) fabrication d'un antisérum selon une des revendications 1 à 4 ; (ii) fabrication d'un matériau de référence d'antigène par un procédé comprenant les étapes de (a) culture de virus de la grippe dans une lignée cellulaire ; (b) purification de virus cultivés dans l'étape (a) ; et (c) inactivation du virus, **caractérisé en ce que** le virus de la grippe utilisé dans l'étape (a) n'a jamais été cultivé dans des oeufs ; et (iii) combinaison des produits des étapes (i) et (ii) dans un kit.

9. Procédé de l'une quelconque des revendications 1 à 8 dans lequel la lignée cellulaire est une lignée cellulaire MDCK.

10. Procédé de la revendication 9 dans lequel la lignée cellulaire MDCK est MDCK33016.
